# EUROPÄISCHE PATENTSCHRIFT

(11) **EP 3 005 963 B1**
(45) Veröffentlichungstag und Bekanntmachung des Hinweises auf die Patenterteilung: **27.03.2019**
(21) Anmeldenummer: 15188347.7
(22) Anmeldetag: 05.10.2015
(51) Int. Cl.: A61B 17/88, A61B 17/16, A61B 17/00, B25B 21/00, B25F 5/00

(54) **ELEKTRISCHE WERKZEUGMASCHINE, INSBESONDERE ELEKTRISCHER SCHRAUBENDREHER FÜR DIE VERWENDUNG IN DER CHIRURGIE**
ELECTRICAL MACHINE TOOL, IN PARTICULAR ELECTRIC SCREW DRIVER FOR USE IN SURGERY
MACHINE-OUTIL ELECTRIQUE, NOTAMMENT TOURNEVIS ELECTRIQUE, A UTILISER EN CHIRURGIE

(30) Priorität: 08.10.2014 EP 14188180
(43) Veröffentlichungstag der Anmeldung: 13.04.2016
(73) Patentinhaber: Medartis Holding AG, 4057 Basel (CH)
(72) Erfinder: Mullis, Andreas, 4456 Tenniken (CH); Huber, Micha, 8409 Winterthur (CH); Künzler, Alfred, 8400 Winterthur (CH); Schmid, Daniel, 3600 Thun (CH)
(74) Vertreter: Hepp Wenger Ryffel AG

(56) Entgegenhaltungen:
- US-A- 5 016 501
- US-A- 5 311 949
- US-B1- 6 220 368

## Beschreibung

Die vorliegende Erfindung befasst sich mit elektrischen Werkzeugmaschinen, insbesondere elektrischen Schraubendrehern, die insbesondere in der Chirurgie verwendet werden können.
Elektrische Schraubendreher haben in der Chirurgie ein breites Anwendungsspektrum. Insbesondere zur Fixierung von Knochenbrüchen und bei Osteotomien werden sie verwendet, um Knochenplatten mit Hilfe von Knochenschrauben zu befestigen oder auch um die Knochenschrauben wieder zu entfernen. Vor allem bei derartigen chirurgischen Anwendungen ist es von Vorteil, wenn der Schraubendreher einhändig bedienbar ist. Dies wird bei einigen bekannten Schraubendrehern durch eine unvorteilhafte Anordnung der Bedienelemente behindert, was im konzentrierten und angespannten OP-Alltag oftmals von erheblichem Nachteil ist.
Auch manuell betätigbare Schraubendreher sind in den genannten Anwendungen unabdingbar, und zwar besonders dann, wenn der Chirurg kurz vor dem Erreichen der endgültigen Schraubenposition auf sein Gefühl und seine Erfahrung angewiesen ist. Ein Wechsel zwischen elektrischem und manuellem Schraubendreher ist allerdings je nach technischer Umsetzung unter Umständen kompliziert und zeitaufwändig, zumal viele übliche Knochenplatten der Fixierung durch eine Vielzahl von Knochenschrauben bedürfen.
Elektrische Schraubendreher in vielen Varianten sind natürlich auch ausserhalb der Chirurgie bekannt. Das Dokument EP 1 066 930 A2 offenbart beispielsweise eine Spindelverriegelung für ein kraftgetriebenes Werkzeug, wie etwa einen Schraubendreher. Diese Spindelverriegelung weist ein erstes Teil auf, das die Ausgangsspindel wenigstens teilweise umgibt und ein erstes Nockenteil umfasst, sowie ein zweites Teil, das ein zweites Nockenteil umfasst. Diese Spindelverriegelung kann in Eingriff mit einem Ausgangszahnrad treten, wodurch das Werkzeug manuell verwendet werden kann. Allerdings gestalten sich das Einschalten dieses Werkzeuges und das Umschalten in den manuellen Modus aufwändig, was insbesondere bei chirurgischen Anwendungen nachteilig ist.

Gegenstand der DE 35 40 652 A1 ist ein elektrischer Schraubendreher mit einer "Ansetz-Einschaltautomatik". Der Motor wird eingeschaltet, wenn eine Druckkraft auf das Werkzeug ausgeübt wird, um eine Schaltstange zu bewegen und einen Schalter für den Motor zu betätigen. Dieser Schraubendreher ist jedoch nicht für das manuelle Einschrauben geeignet.

Das Dokument GB 853,407 offenbart einen elektrischen Schraubendreher mit einer Antriebsspindel, die einen Körper mit kreuzförmigem Querschnitt aufweist. Mit Hilfe eines seitlich angeordneten Druckknopfes kann durch radialen Druck ein Käfig zwischen zwei Positionen bewegt werden: In der ersten Position arretiert der Käfig die Antriebswelle und öffnet gleichzeitig einen Kontakt, sodass kein Antrieb erfolgt. In der zweiten Position ist die Antriebswelle freigegeben, und der Kontakt wird geschlossen. Das Betätigen des Druckknopfes erfordert also einen zusätzlichen Bedienschritt, was in Praxis (vor allem während einer Operation) sehr umständlich ist.

Die in US 4,804,048 offenbarte Werkzeugmaschine verfügt über einen Stellring. Durch axiales Verschieben dieses Stellringes erfolgt eine Arretierung zwischen Werkzeug und Gehäuse, sodass das Werkzeug als manueller Schraubendreher verwendet werden kann. Auch hier stellt das Betätigen des Stellringes jedoch einen zusätzlichen Bedienschritt dar, für den der Benutzer überdies beide Hände benötigt. Abgesehen davon ist diese Werkzeugmaschine aufgrund ihrer Formgebung und Dimensionen für die chirurgische Verwendung nicht geeignet.

Das Dokument WO 03/011533 A2 offenbart ebenfalls ein Werkzeug mit einem Stellring, der es erlaubt, das Werkzeug in einem manuellen Modus zu betreiben, in welchem auf das Gehäuse einwirkende Kräfte direkt auf die Spindel übertragen werden. Auch hier erfordert die Betätigung des Stellringes einen weiteren Bedienschritt dar, für den der Benutzer beide Hände benötigt.

In DE 1 478 828 A1 ist ein motorisch angetriebenes Schraubgerät mit einer Spindel und einem Gehäuse offenbart, wobei diese beiden Teile durch eine Kupplung derart miteinander verbunden sind, dass sie sich bei manuellem Antrieb im Schraubendrehsinn im Eingriff, dagegen bei manuellem Antrieb entgegen dem Schraubendrehsinn ausser Eingriff befinden. Eine einhändige Bedienung erlaubt aber auch dieses Schraubgerät nicht.

Das Dokument EP 0 088 836 A1 offenbart einen elektrisch angetriebenen Schraubendreher mit einem Ratschen-Mechanismus. Dieser erlaubt ein Einschrauben, welches mechanisch und elektrisch erfolgen kann, sowie ein festes Anziehen, welches manuell erfolgen kann. Der Motor muss jedoch mittels eines separaten Schalters eingeschaltet werden, was die Bedienung unkomfortabel macht.

Das Dokument US 6,220,368 offenbart einen elektrisch antreibbaren chirurgischen Schraubendreher mit einem Schieber. In einer ersten Position des Schiebers wird der Schraubendreher von einem elektrischen Motor angetrieben. In einer zweiten Position des Schiebers kann der Schraubendreher manuell bedient werden. Dabei wird der Schieber bedient, indem er manuell vor (Motorantrieb) oder zurück (Handantrieb) geschoben wird und dann in der Position verbleibt. Die Bedienung auch dieses Schraubendrehers ist daher recht aufwändig, da zum Wechseln vom Handantrieb in den Motorantrieb der Schieber mit einer separaten Handbewegung bedient werden muss.

Angesichts dieser dem Stand der Technik innewohnenden Nachteile besteht die Aufgabe der vorliegenden Erfindung in der Bereitstellung einer elektrischen Werkzeugmaschine, insbesondere eines elektrischen Schraubendrehers, die eine erleichterte Bedienung erlaubt. Insbesondere soll die Werkzeugmaschine auf möglichst einfache und natürliche Weise eingeschaltet werden können, ohne dass schwer zu erreichende Bedienelemente betätigt werden müssen. Zudem soll die Werkzeugmaschine in möglichst einfacher und natürlicher Art wahlweise in einem motorgetriebenen Modus oder einem manuellen Modus betrieben werden können. Insbesondere für eine Verwendung in der Chirurgie sollen das Einschalten des Motors und das Umschalten zwischen motorgetriebenem und manuellem Modus möglichst einhändig erfolgen können. Weiterhin soll die Werkzeugmaschine möglichst wenige Bedienelemente aufweisen.

Diese Aufgaben werden gelöst durch eine elektrische Werkzeugmaschine, die Folgendes enthält:
- ein Gehäuse;
- ein Rotationswerkzeug oder eine Werkzeugaufnahme zum insbesondere lösbaren Aufnehmen eines Rotationswerkzeugs;
- einen Elektromotor zum Antreiben des Rotationswerkzeugs bzw. der Werkzeugaufnahme;
- mindestens einen Schalter, welcher derart ausgebildet und angeordnet ist, dass das Rotationswerkzeug bzw. die Werkzeugaufnahme nur dann vom Elektromotor antreibbar ist, wenn eine auf ein Betätigungselement der Werkzeugmaschine einwirkende axiale Druckkraft einen vorgegebenen zweiten Schwellenwert überschreitet.

Bei der elektrischen Werkzeugmaschine kann es sich beispielsweise um einen elektrischen Schraubendreher handeln. Entsprechend kann das Rotationswerkzeug eine Schraubendreherklinge sein. Eine Werkzeugmaschine mit einer Werkzeugaufnahme kann wahlweise mit verschiedenen Rotationswerkzeugen verwendet werden, die abwechselnd von der Werkzeugaufnahme aufgenommen werden. Alternativ kann die Werkzeugmaschine bereits selbst das Rotationswerkzeug enthalten, welches unlösbar befestig sein kann, so dass die Werkzeugmaschine beispielsweise als Wegwerfwerkzeug zur einmaligen Verwendung ausgebildet sein kann.

Als Schalter wird hier und im Folgenden ein Bauelement verstanden, welches wahlweise in einen Einschaltzustand und einen Ausschaltzustand bringbar ist, wobei das Rotationswerkzeug bzw. die Werkzeugaufnahme nur dann vom Elektromotor antreibbar ist, wenn sich der Schalter im Einschaltzustand befindet, nicht aber auch, wenn er sich im Ausschaltzustand befindet. Der Schalter kann innerhalb eines Gehäuses der Werkzeugmaschine oder an einem Gehäuse der Werkzeugmaschine angeordnet sein. Er kann mindestens einen Schaltkontakt und mindestens einen Gegenkontakt enthalten, welcher beispielsweise als Kontaktstift ausgebildet sein kann. Der Schaltkontakt kann im Einschaltzustand in Kontakt mit dem mindestens einen Gegenkontakt sein und im Ausschaltzustand nicht in Kontakt mit dem mindestens einen Gegenkontakt sein.

Durch Anwendung einer axialen Druckkraft auf das Betätigungselement kann also der Schalter betätigt werden und dafür sorgen, dass das Rotationswerkzeug bzw. die Werkzeugaufnahme vom Elektromotor angetrieben wird. Zum Antreiben kann also auf das umständliche Bewegen eines Schiebers verzichtet werden, so wie es beispielsweise beim Schraubendreher gemäss US 6,220,368 erforderlich ist.

Die elektrische Werkzeugmaschine, insbesondere der elektrische Schraubendreher, kann für die Verwendung in der Chirurgie vorgesehen und ausgebildet sein, beispielsweise für das Eindrehen und/oder Ausdrehen von Knochenschrauben. Selbstverständlich kann die elektrische Werkzeugmaschine natürlich auch für andere als für chirurgische Verwendungen ausgebildet sein.

Die erfindungsgemässe Werkzeugmaschine ist gekennzeichnet durch Sperrmittel, welche in eine Sperrposition und in eine Freigabeposition bringbar sind und derart ausgebildet und angeordnet sind, dass das Rotationswerkzeug bzw. die Werkzeugaufnahme
- in der Sperrposition gegenüber dem Gehäuse gesperrt oder nur bei Überschreiten eines vorgegebenen Drehmoments relativ zum Gehäuse beweglich ist, wenn eine auf das Betätigungselement einwirkende axiale Druckkraft einen vorgegebenen ersten Schwellenwert unterschreitet, und
- in der Freigabeposition gegenüber dem Gehäuse beweglich ist, wenn die auf das Betätigungselement einwirkende axiale Drucckraft den vorgegebenen ersten Schwellenwert überschreitet.

In Abhängigkeit von der auf das Betätigungselement einwirkenden axialen Druckkraft befindet sich das Rotationswerkzeug bzw. die Werkzeugaufnahme also entweder in der Sperrposition oder in der Freigabeposition. In der Sperrposition kann die Werkzeugmaschine in einem manuellen Modus bedient werden, in dem das Gehäuse der Werkzeugmaschine oder ein anderes, gegenüber dem Gehäuse nicht drehbares Bauteil mit der Hand ergriffen und gedreht wird.

Dabei kann in der Sperrposition in einer ersten Variante eine vollständige Sperrung zwischen Rotationswerkzeug bzw. Werkzeugaufnahme und Gehäuse vorliegen. In einer zweiten Variante ist das Rotationswerkzeug bzw. die Werkzeugaufnahme nur bei Überschreiten eines vorgegebenen Drehmoments relativ zum Gehäuse beweglich. Diese zweite Variante kann beispielsweise mit Hilfe einer Drehmomentkupplung realisiert werden. Diese erlaubt ein manuelles Drehen des Rotationswerkzeugs bzw. der Werkzeugaufnahme, solange das vorgegebene Drehmoment unterschritten ist. Wird dieses vorgegebene Drehmoment überschritten, so wird die Sperre zwischen Rotationswerkzeug bzw. Werkzeugaufnahme und Gehäuse gelöst. Insgesamt kann in dieser zweiten Variante also beispielsweise ein Eindrehen einer Knochenschraube nur bis zu dem vorgegebenen Drehmoment ermöglicht werden.

Da in der Freigabeposition das Rotationswerkzeug bzw. die Werkzeugaufnahme gegenüber dem Gehäuse beweglich ist, ist das Rotationswerkzeug bzw. die Werkzeugaufnahme in dieser Position vom Elektromotor antreibbar, ohne dass dies durch eine Sperrung zwischen Rotationswerkzeug bzw. Werkzeugaufnahme und Gehäuse verhindert wird.

Die axiale Druckkraft bestimmt erfindungsgemäss also sowohl die Betätigung des Schalters, der für das Antreiben mittels des Elektromotors verantwortlich ist, als auch das Sperren oder Freigeben. Dies erlaubt eine deutlich erleichterte Bedienung der Werkzeugmaschine. Denn insbesondere ist auch für das Wechseln zwischen Sperrposition und Freigabeposition keine umständliche Bewegung eines Schiebers wie in US 6,220,368 erforderlich. Weiterhin kann die Werkzeugmaschine in einfacher und natürlicher Art wahlweise in einem motorgetriebenen Modus oder einem manuellen Modus betrieben werden, wobei insbesondere ein einhändiges Umschalten ermöglicht wird. Zudem reduziert die erfindungsgemässe Ausbildung die Anzahl der nötigen Bedienelemente.

Dabei kann die axiale Druckkraft im Wesentlichen in Richtung auf das Rotationswerkzeug bzw. die Werkzeugaufnahme hin wirken. Alternativ kann die axiale Druckkraft im Wesentlichen in Richtung vom Rotationswerkzeug bzw. von der Werkzeugaufnahme weg wirken.

In einigen Ausführungsformen ist das Betätigungselement derart ausgebildet, dass es sich bei Einwirken einer axialen Druckkraft insgesamt gegenüber einem Gehäuse der Werkzeugmaschine verschiebt. In anderen Ausführungsformen kann das Betätigungselement aber beispielsweise auch derart elastisch ausgebildet sein, dass es sich bei Einwirken einer axialen Druckkraft verformt.

Es ist denkbar und liegt im Rahmen der Erfindung, dass das Betätigungselement durch das Rotationswerkzeug bzw. die Werkzeugaufnahme gebildet ist. In dieser Variante kann die axiale Drucckraft aufgebracht werden, indem das Rotationswerkzeug bzw. die Werkzeugaufnahme in axialer Richtung gegen einen Gegenstand gedrückt wird, beispielsweise gegen eine einzudrehende oder auszudrehende Knochenschraube. Die Werkzeugmaschine kann in dieser Variante an jeder Stelle des Gehäuses gehalten werden. Allerdings erfordert diese Variante eine axiale Verschiebbarkeit und Vorspannung des Rotationswerkzeuges bzw. der Werkzeugaufnahme.

Bevorzugt ist es daher, wenn das Betätigungselement durch ein Schaltelement gebildet wird, welches gegenüber dem Gehäuse in axialer Richtung verschiebbar ist, also entlang einer Drehachse des Rotationswerkezeuges bzw. der Werkzeugaufnahme. Ein solches Schaltelement kann auch weitere Bedienfunktionen bereitstellen, wie weiter unten noch näher erläutert wird.

Weiterhin bevorzugt ist das Schaltelement in axialer Richtung vorgespannt, und zwar bevorzugt in einer vom Rotationswerkzeug bzw. von der Werkzeugaufnahme weg weisenden Richtung. Der erste und der zweite Schwellenwert der axialen Druckkraft können dann nur erreicht werden, wenn diese Vorspannung in entsprechendem Masse überwunden wird. Die genannte Vorspannung kann beispielsweise mittels mindestens einer Feder erreicht werden, insbesondere mittels mindestens einer Druckfeder. Hierdurch entsteht ein Zusammenhang zwischen der Position des Schaltelementes und der axialen Druckkraft, die das Sperren oder Freigeben und das Antreiben mittels des Elektromotors bestimmt.

Die axiale Verschiebbarkeit des Schaltelementes ist besonders dann vorteilhaft, wenn das Schaltelement derart ausgebildet und angeordnet ist, dass die Werkzeugmaschine von einem Benutzer durch insbesondere einhändiges Halten nur am Schaltelement bestimmungsgemäss bedienbar ist. Denn dann kann die axiale Drucckraft dadurch aufgebracht werden, dass der Benutzer die Werkzeugmaschine nur am Schaltelement hält und das Rotationswerkzeug bzw. die Werkzeugaufnahme in axialer Richtung gegen einen Gegenstand drückt, beispielsweise gegen eine einzudrehende oder auszudrehende Knochenschraube. Hält der Benutzer die Werkzeugmaschine hingegen am Gehäuse, nicht aber auch am Schaltelement, so kann er eine axiale Druckkraft auf die Werkzeugmaschine und damit auch auf das Rotationswerkzeug bzw. die Werkzeugaufnahme ausüben, ohne dass hierdurch die auf das Schaltelement einwirkende axiale Druckkraft den ersten Schwellenwert überschreitet und somit die Freigabeposition erreicht wird oder sogar das Rotationswerkzeug bzw. die Werkzeugaufnahme vom Elektromotor angetrieben wird. Dies ist vor allem für den manuellen Modus besonders vorteilhaft.

Vorzugsweise sind die Sperrmittel derart vorgespannt, dass sie sich bei Unterschreiten des ersten Schwellenwertes in der Sperrposition befinden. Durch entsprechende Wahl einer solchen Vorspannung kann der erste Schwellenwert der axialen Druckkraft beeinflusst werden. Diese Vorspannung kann mittels mindestens einer Feder bewirkt werden, insbesondere mittels mindestens einer Druckfeder und/oder mittels mindestens einer Zugfeder.

Vorteilhafterweise ist der Elektromotor mindestens in der Freigabeposition mit dem Rotationswerkzeug bzw. der Werkzeugaufnahme gekoppelt, um das Rotationswerkzeug bzw. die Werkzeugaufnahme anzutreiben. Diese Kopplung kann mindestens ein Getriebe und/oder mindestens eine Kupplung enthalten. Die Kopplung zwischen Elektromotor und Rotationswerkzeug bzw. die Werkzeugaufnahme kann auch bestehen, wenn das Rotationswerkzeug bzw. die Werkzeugaufnahme gerade nicht vom Elektromotor angetrieben wird.

Mit besonderem Vorteil ist der Elektromotor nur in der Freigabeposition, nicht aber auch in der Sperrposition, eingeschaltet (beispielsweise aufgrund einer Verbindung mit einer Energiequelle), um das Rotationswerkzeug bzw. die Werkzeugaufnahme anzutreiben. Es ist ferner auch denkbar und liegt im Rahmen der Erfindung, dass der Elektromotor auch in der Sperrposition eingeschaltet ist, wobei er aber dann nicht derart mit dem Rotationswerkzeug bzw. der Werkzeugaufnahme gekoppelt ist, um es/sie anzutreiben.

Die Werkzeugmaschine weist vorzugsweise ein mit dem Betätigungselement verbundenes Schaltrohr auf, das bei Einwirken einer axialen Druckkraft auf das Betätigungselement gegenüber dem Gehäuse in axialer Richtung verschiebbar ist und nur bei Überschreiten des ersten Schwellenwertes derart mit den Sperrmitteln zusammenwirkt, dass diese sich in der Freigabeposition befinden. Vorzugsweise ist das Schaltrohr starr mit dem Betätigungselement verbunden, beispielsweise mit Hilfe einer oder mehrerer Schrauben. Die zweiteilige Ausführung von Betätigungselement und Schaltrohr hat den Vorteil, dass das Betätigungselement an der Aussenseite der Werkzeugmaschine angeordnet werden kann, wohingegen das Schaltrohr sich im Inneren der Werkzeugmaschine befinden kann.

In bevorzugten Ausführungsformen enthalten die Sperrmittel mindestens einen Sperrhebel mit einem ersten Hebelarm und einem zweiten Hebelarm. Bevorzugt enthalten die Sperrmittel mindestens zwei und besonders bevorzugt genau zwei Sperrhebel. Dabei wirkt der Sperrhebel derart mit dem Schaltrohr zusammen, dass der Sperrhebel nur bei Unterschreiten des ersten Schwellenwertes durch Eingriff des ersten Hebelarmes mit dem Schaltrohr in die Sperrposition bewegbar ist. In dieser Sperrposition ist der zweite Hebelarm mit mindestens einem Nocken einer Antriebswelle der Werkzeugmaschine in Eingriff, die den Elektromotor mit dem Rotationswerkzeug bzw. der Werkzeugaufnahme verbindet. Insbesondere kann am zweiten Hebelarm ein sich in radialer Richtung nach innen erstreckender Fortsatz angeformt sein, der in der Sperrposition in einen zwischen zwei Nocken der Antriebswelle gebildeten Zwischenraum eindringt. Weiterhin bevorzugt ist der Sperrhebel um eine Schwenkachse schwenkbar, welche im Wesentlichen parallel zur Drehachse des Rotationswerkzeugs bzw. der Werkzeugaufnahme verläuft.

Durch einen solchen Aufbau kann eine axiale Bewegung des Schaltrohres in eine Schwenkbewegung in einer im Wesentlichen senkrecht zur Drehachse verlaufenden Ebene umgesetzt werden. Ein solcher Aufbau ist besonders Platz sparend.

Dabei ist es möglich, dass zwischen Elektromotor und Antriebswelle und/oder zwischen Antriebswelle und Rotationswerkzeug bzw. Werkzeugaufnahme mindestens ein Getriebe und/oder mindestens eine Kupplung vorliegt, insbesondere mindestens eine Drehmomentkupplung.

Besonders vorteilhaft ist es, wenn das Schaltrohr eine erste Kontaktfläche, insbesondere eine erste Rampe, aufweist und der erste Hebelarm eine zweite Kontaktfläche, insbesondere eine zweite Rampe, aufweist, welche derart ausgebildet und angeordnet sind, dass der erste Hebelarm bei Überschreiten des ersten Schwellenwertes durch Kontakt der zweiten Kontaktfläche mit der ersten Kontaktfläche in radiale Richtung bewegt wird. Auch diese Weiterbildung trägt zu einer Platz sparenden Bauweise bei. In vorteilhaften Ausgestaltungen hat die erste Kontaktfläche die Form eines partiellen Kegelmantels. Die Kontaktflächen müssen allerdings nicht zwingend beide als Rampen ausgebildet sein. Beispielsweise kann nur die erste Kontaktfläche als Rampe ausgebildet sein und die zweite Kontaktfläche als Ecke, wobei die Rampe und die Ecke derart ausgebildet und angeordnet sind, dass der erste Hebelarm bei Überschreiten des ersten Schwellenwertes durch Kontakt der Ecke mit der Rampe in radiale Richtung bewegt wird. Denkbar ist natürlich auch eine umgekehrte Ausführung, in der nur die zweite Kontaktfläche als Rampe ausgebildet ist und die erste Kontaktfläche beispielsweise als Ecke, wobei die Rampe und die Ecke derart ausgebildet und angeordnet sind, dass der erste Hebelarm bei Überschreiten des ersten Schwellenwertes durch Kontakt der Rampe mit der Ecke in radiale Richtung bewegt wird. Anstelle der Ecke kann in beiden Varianten beispielsweise auch eine sphärische Teilfläche vorgesehen sein.

Zur Erzeugung der oben bereits erwähnten Vorspannung der Sperrmittel können zwei Sperrhebel vorgesehen sein, deren zweite Hebelarme beispielsweise durch eine Feder miteinander verbunden sind, insbesondere durch eine Zugfeder, eine Biegefeder oder eine Druckfeder.

In vorteilhaften Ausgestaltungen ist der Elektromotor und/oder eine den Elektromotor mit dem Rotationswerkzeug bzw. der Werkzeugaufnahme verbindende Kupplung und/oder ein den Elektromotor mit dem Rotationswerkzeug bzw. der Werkzeugaufnahme verbindendes Getriebe derart ausgebildet und/oder angeordnet, dass das Rotationswerkzeug bzw. die Werkzeugaufnahme wahlweise in einer von zwei zueinander entgegengesetzten Drehrichtungen antreibbar ist. Dies ermöglicht es beispielsweise, mit ein und derselben Werkzeugmaschine Schrauben, insbesondere Knochenschrauben, einzudrehen und auch bei Bedarf wieder auszudrehen.

Eine bevorzugte Weiterbildung sieht vor, dass die Werkzeugmaschine ein Schaltelement aufweist, mit dessen Hilfe mindestens ein Antriebsparameter der Werkzeugmaschine auswählbar ist. Dabei ist das Schaltelement relativ zum Gehäuse um eine Einstellachse verdrehbar, welche im Wesentlichen parallel zur Drehachse des Rotationswerkzeugs bzw. der Werkzeugaufnahme verläuft oder mit dieser Drehachse übereinstimmt. Bei dem Antriebsparameter kann es sich insbesondere um die Drehrichtung und/oder die Drehgeschwindigkeit des Rotationswerkzeuges bzw. der Werkzeugaufnahme handeln. Eine solche Verdrehbarkeit um die genannte Drehachse erleichtert die Auswahl des Antriebsparameters.

Mit besonderem Vorteil ist das genannte Schaltelement identisch mit dem oben beschriebenen Schaltelement, welches das Betätigungselement bildet und gegenüber dem Gehäuse in axialer Richtung verschiebbar ist. Auf diese Weise kann durch ein und dasselbe Schaltelement zwischen Sperrposition und Freigabeposition gewechselt werden, der Schalter kann betätigt werden, und der Antriebsparameter, insbesondere die Drehrichtung, kann ausgewählt werden.

Vorzugsweise ist das Schaltelement derart ausgebildet und angeordnet, dass es nur dann relativ zum Gehäuse verdrehbar ist, wenn die auf das Betätigungselement einwirkende axiale Drucckraft den ersten Schwellenwert unterschreitet. Mit anderen Worten kann der Antriebsparameter nur dann verändert werden, wenn die Sperrposition vorliegt. Wenn hingegen der erste Schwellenwert überschritten wird und folglich die Freigabeposition vorliegt, ist das Schaltelement nicht mehr verdrehbar, sodass der Antriebsparameter nicht verändert werden kann. Durch diese Massnahme kann beispielsweise verhindert werden, dass die Drehrichtung umgestellt wird, während das Rotationswerkzeug bzw. die Werkzeugaufnahme angetrieben wird.

In einigen Ausführungsformen enthält der Schalter mindestens einen Schaltkontakt und mindestens einen, bevorzugt mindestens zwei, besonders bevorzugt drei Gegenkontakte, die beispielsweise als Kontaktstifte ausgebildet sein können. Der Schaltkontakt kann (beispielsweise in Abhängigkeit von der Stellung des Schaltelementes) mit dem Gegenkontakt (insbesondere dem Kontaktstift) bzw. mit einem oder mehreren der Gegenkontante (insbesondere einem oder mehreren der Kontaktstifte) verbindbar sein.

In einer möglichen Ausgestaltung enthält der Schalter einen insbesondere kreisbogenförmigen Schaltkontakt und mindestens drei Kontaktstifte, wobei der Schaltkontakt bei Überschreiten des zweiten Schwellenwertes
- in einer ersten Stellung des Schaltelementes einen ersten der Kontaktstifte mit einem zweiten der Kontaktstifte verbindet, so dass das Rotationswerkzeug bzw. die Werkzeugaufnahme in einer ersten Drehrichtung antreibbar ist, und
- in einer zweiten Stellung des Schaltelementes den ersten der Kontaktstifte, den zweiten der Kontaktstifte und einen dritten der Kontaktstifte miteinander verbindet, so dass das Rotationswerkzeug bzw. die Werkzeugaufnahme in einer zweiten Drehrichtung antreibbar ist, welche zur ersten Drehrichtung entgegengesetzt ist.

In Abhängigkeit von dem verwendeten Elektromotor können der Schaltkontakt und die Kontaktstifte aber auch ausgebildet sein, um andere und/oder zusätzliche Stellungen einzunehmen. Insbesondere ist es beispielsweise auch denkbar und liegt im Rahmen der Erfindung, dass der Schalter mehr als drei Kontaktstifte und/oder mehr als einen Schaltkontakt enthält. Hierdurch können verschiedene Geschwindigkeiten oder weitere Betriebsparameter eingestellt werden.

Von besonderem Vorteil ist es, wenn der Schalter derart ausgebildet und angeordnet ist, dass der Elektromotor mittels des Schalters eingeschaltet wird, wenn eine auf das Betätigungselement einwirkende axiale Druckkraft den zweiten Schwellenwert überschreitet. Wie oben bereits angedeutet wurde, ist es alternativ auch denkbar, dass der Elektromotor auch bei Unterschreiten des zweiten Schwellenwertes eingeschaltet ist, solange der Elektromotor dann nicht mit dem Rotationswerkzeug bzw. der Werkzeugaufnahme gekoppelt ist, um das Rotationswerkzeug bzw. die Werkzeugaufnahme anzutreiben.

Besonders vorteilhaft ist es ebenfalls, wenn der erste Schwellenwert kleiner ist als der zweite Schwellenwert. Auf diese Weise wird sichergestellt, dass bei ansteigender axialer Druckkraft zunächst die Freigabeposition erreicht wird und erst anschliessend das Rotationswerkzeug bzw. die Werkzeugaufnahme vom Elektromotor angetrieben wird.

Im Folgenden wird die Erfindung anhand eines Ausführungsbeispiels und mehrerer Zeichnungen im Detail erläutert. Dabei zeigen
- Figur 1:: eine Seitenansicht eines erfindungsgemässen elektrischen Schraubendrehers in einer Ruheposition;
- Figur 2:: eine Vorderansicht auf den erfindungsgemässen Schraubendreher in der Ruheposition mit Darstellung der Sperrhebel als verdeckte Kanten;
- Figur 3:: eine Schnittansicht entlang der Linie C-C in Figur 2;
- Figur 4:: eine Schnittansicht entlang der Linie A-A in Figur 2;
- Figur 5:: eine Schnittansicht entlang der Linie D-D in Figur 4;
- Figur 6:: das Schaltrohr des erfindungsgemässen Schraubendrehers;
- Figur 7:: den Sperrhebel des erfindungsgemässen Schraubendrehers;
- Figur 8:: eine Schnittansicht entlang der Linie H-H in Figur 4;
- Figur 9:: eine Seitenansicht des erfindungsgemässen Schraubendrehers in einer Aktivposition;
- Figur 10:: eine Vorderansicht auf den erfindungsgemässen Schraubendreher in der Aktivposition mit Darstellung der Sperrhebel als verdeckte Kanten;
- Figur 11:: eine Schnittansicht entlang der Linie B-B in Figur 10;
- Figur 12:: eine Schnittansicht entlang der Linie E-E in Figur 11;
- Figur 13:: eine Schnittansicht entlang der Linie F-F in Figur 3;
- Figur 14:: eine Schnittansicht entlang der Linie G-G in Figur 3;
- Figur 15:: eine Schnittansicht entlang der Linie I-I in Figur 11.

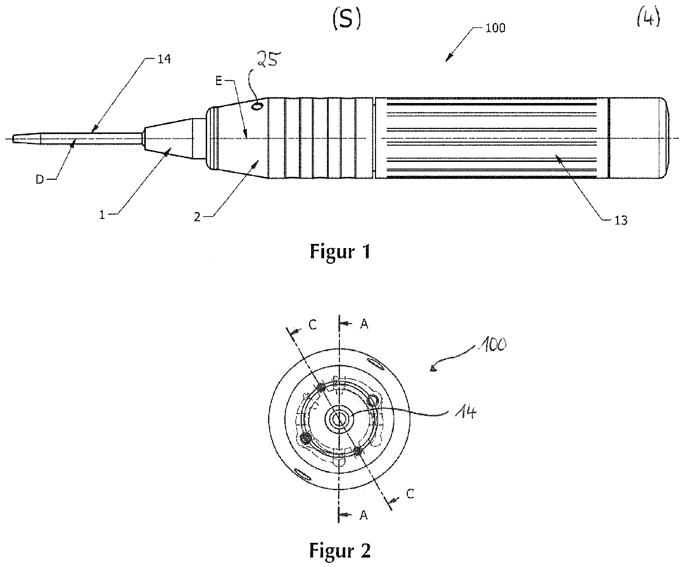

Figur 1 zeigt einen erfindungsgemässen elektrischen Schraubendreher 100 in einer Seitenansicht. Dieser enthält ein Gehäuse 13 und eine Werkzeugaufnahme 1, in dem ein als Schraubendreherklinge 14 ausgebildetes Rotationswerkzeug aufgenommen ist. Mittels eines hier nicht erkennbaren Elektromotors ist die Schraubendreherklinge 14 um eine Drehachse D rotierend antreibbar. Dieser Schraubendreher 100 kann in der Chirurgie verwendet werden, beispielsweise um Knochenschrauben durch Öffnungen einer Knochenplatte hindurch in einen Knochen hineinzudrehen und/oder aus diesem herauszudrehen.

Der Schraubendreher 100 verfügt ferner über ein Schaltelement 2, welches gegenüber dem Gehäuse 13 in axialer Richtung, also entlang der Drehachse D, verschiebbar ist. Zudem ist das Schaltelement 2 relativ zum Gehäuse 13 um eine Einstellachse E verdrehbar, welche mit der Drehachse D übereinstimmt. Der Schraubendreher 100 ist von einem Benutzer, beispielsweise einem Chirurgen, durch einhändiges Halten nur am Schaltelement 2 bedienbar. Mittels zweier Verschlussschrauben 25, von denen hier nur eine erkennbar ist, ist das Schaltelement 2 mit einem hier nicht erkennbaren Schaltrohr und auch mit einem hier nicht erkennbaren Schaltring starr verbunden.

In Figur 1 befindet sich der Schraubendreher 100 in einer Ruheposition 4, in der die Schraubendreherklinge 14 nicht vom Elektromotor angetrieben wird und in der zudem eine Sperrposition S vorliegt, in der die Schraubendreherklinge 14 gegenüber dem Gehäuse 13 gesperrt ist. In dieser Sperrposition S kann der Schraubendreher 100 in einem manuellen Modus bedient werden, indem das Gehäuse 13 mit der Hand ergriffen und gedreht wird.

In Figur 2 ist der Schraubendreher 100 in einer Vorderansicht in Richtung auf die Schraubendreherklinge 14 dargestellt. Dabei sind die Sperrhebel als verdeckte Kanten dargestellt.

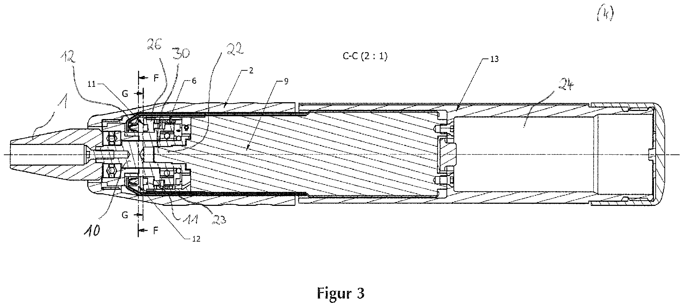

In Figur 3 ist eine Schnittansicht entlang der Linie C-C in Figur 2 wiedergegeben. Im Inneren des Schraubendrehers 100 befindet sich ein Elektromotor 9, der durch eine hier nicht dargestellte Energiequelle, wie beispielsweise eine Batterie, mit Energie versorgt werden kann, die in einem Aufnahmefach 24 aufgenommen werden kann. Ein sechskantförmig ausgebildetes Ende 30 einer Motorwelle 22 des Elektromotors 9 greift in eine sechskantförmige Aufnahmeöffnung einer Antriebswelle 10 ein. Die Antriebswelle 10 ist ihrerseits mit der Werkzeugaufnahme 1 verbunden. Auf diese Weise kann die Werkzeugaufnahme 1 vom Elektromotor 9 rotierend angetrieben werden.

Das Schaltelement 2 ist mittels einer Druckfeder 6 in axialer Richtung vorgespannt, und zwar in einer von der Werkzeugaufnahme 1 weg weisenden Richtung. Wenn ein Benutzer, beispielsweise ein Chirurg, den Schraubendreher 100 nur am Schaltelement 2 hält und die Schraubendreherklinge 14 gegen einen Gegenstand drückt, beispielsweise gegen eine einzudrehende oder auszudrehende Knochenschraube, so bewegt sich das Schaltelement 2 gegen die durch die Druckfeder 6 hervorgerufene Vorspannung in Richtung auf die Werkzeugaufnahme 1 zu. Aufgrund dieser axialen Verschiebung des Schaltelementes gegenüber dem Gehäuse 13 wird beim Überschreiten eines vorgegebenen ersten Schwellenwertes der axialen Druckkraft zunächst die Werkzeugaufnahme 1 gegenüber dem Gehäuse 13 freigegeben, und anschliessend wird beim Überschreiten auch eines vorgegebenen zweiten, grösseren Schwellenwertes der axialen Drucckraft der Elektromotor 9 eingeschaltet, wie im Folgenden noch detailliert erläutert wird.

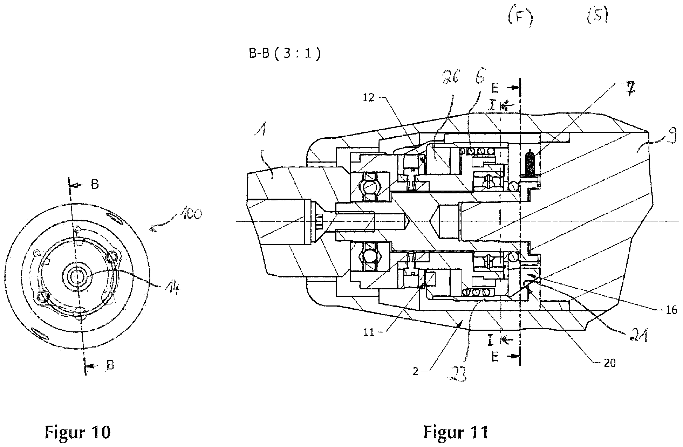

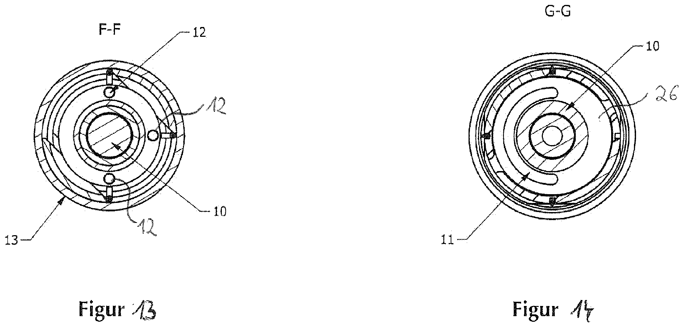

In Figur 3 sind ebenfalls ein kreisbogenförmiger Schaltkontakt 11 und zwei von drei Kontaktstiften 12 erkennbar, die zusammen einen Schalter bilden, mittels dessen der Elektromotor 9 eingeschaltet werden kann (siehe dazu auch Figur 11). Wie im Zusammenhang mit den Figuren 13 und 14 noch detailliert beschrieben wird, ist der Schaltkontakt 11 in dem bereits erwähnten Schaltring 26 aufgenommen. Insbesondere ist der hier dargestellte Schalter innerhalb des Gehäuses 13 angeordnet. In anderen erfindungsgemässen Ausführungsformen ist es natürlich ebenso denkbar, dass ein Schalter an einem Gehäuse des Schraubendrehers angeordnet ist. Ebenfalls ist es in anderen erfindungsgemässen Ausführungsbeispielen denkbar, dass der Schalter nur einen einzigen Kontaktstift enthält.

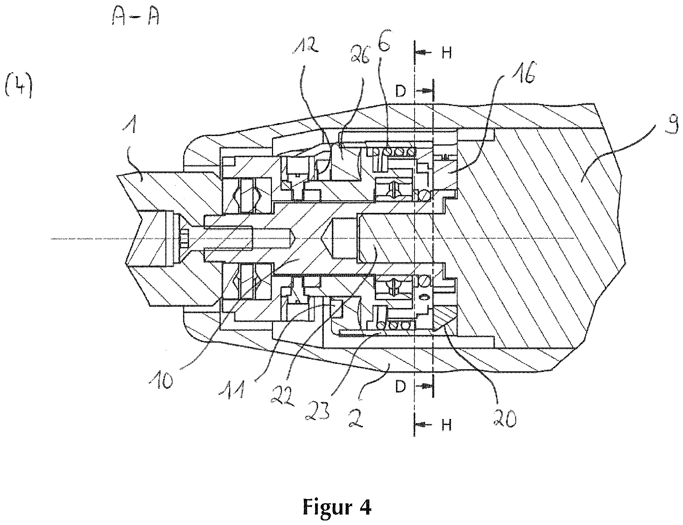

Figur 4, die eine Schnittansicht entlang der Linie A-A in Figur 2 darstellt, zeigt im Detail die Motorwelle 22, die Antriebswelle 10, die Druckfeder 6, den kreisbogenförmigen Schaltkontakt 11 und einen der drei Kontaktstifte 12. Durch axiale Verschiebung des Schaltelementes 2 werden aufgrund der Verschlussschrauben 25 auch das Schaltrohr 23 und der Schaltring 26 in axialer Richtung auf die Werkzeugaufnahme 1 zu bewegt. Auf diese Weise wird auch der im Schaltring 26 enthaltene Schaltkontakt 11 in Richtung auf die Kontaktstifte 12 zu bewegt und gerät mit diesen in Kontakt, wenn die auf das Schaltelement 2 einwirkende axiale Druckkraft den zweiten Schwellenwert überschreitet. Der zweite Schwellenwert kann durch entsprechende Wahl der Dimensionen und der Federkonstante der Druckfeder 6 eingestellt werden. Je nach Dreheinstellung des Schaltelementes 2 gerät der Schaltkontakt 11 dabei in Kontakt mit zweien oder dreien der Kontaktstifte 12, so dass der Elektromotor 9 eingeschaltet wird und damit die Schraubendreherklinge 14 angetrieben wird, wobei die Drehrichtung durch die Dreheinstellung des Schaltelementes 2 bestimmt wird.

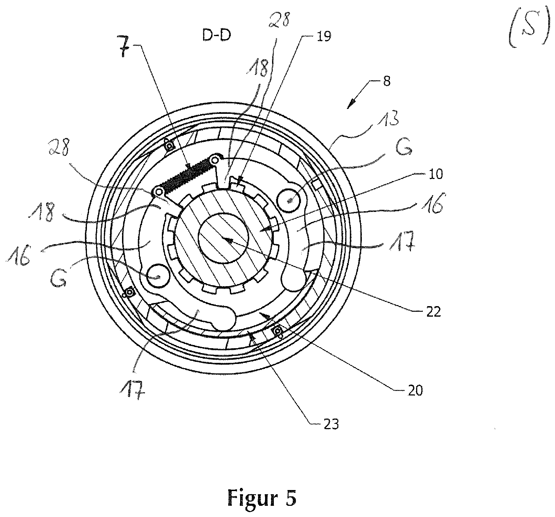

Weiterhin sind in Figur 4 einer von zwei Sperrhebeln 16 und eine erste Rampe 20 des Schaltohres 23 erkennbar, deren Funktion im Folgenden im Zusammenhang mit Figur 5 erläutert wird.

Diese Figur 5 zeigt eine Schnittansicht entlang der Linie D-D in Figur 4. Durch die Drehachse D verläuft ein zylinderförmiger Abschnitt der Motorwelle 22, der innerhalb der Antriebswelle 10 verläuft. Die Sperrmittel 8 enthalten zwei Sperrhebel 16 mit jeweils einem ersten Hebelarm 17 und einem zweiten Hebelarm 18. Die Sperrhebel 16 sind um jeweilige Schwenkachsen G schwenkbar, die parallel zur Drehachse D der Werkzeugaufnahme 1 vorlaufen, aber zu dieser versetzt sind. Mittels einer Zugfeder 7 sind die beiden zweiten Hebelarmen 18 gegeneinander vorgespannt, so dass sich die Sperrhebel 16 bei Unterschreiten des ersten Schwellenwertes in der in Figur 5 gezeigten Sperrposition S befinden.

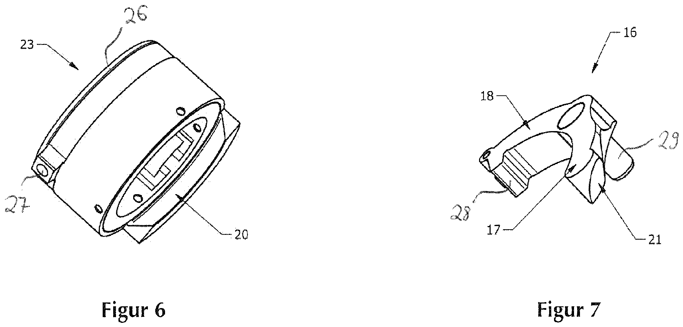

An ihren ersten Hebelarmen 17 weisen die Sperrhebel 16 jeweils eine zweite Kontaktfläche 21 auf, die als zweite Rampe 21 ausgebildet sind (siehe dazu auch Figur 7). Entsprechend weist das Schaltrohr 23 eine erste Kontaktfläche 20 auf, die eine erste Rampe 20 bildet, welche die Form eines partiellen Kegelmantels aufweist (siehe Figur 6). Weiterhin weisen die beiden zweiten Hebelarme 18 jeweils einen sich in radialer Richtung nach innen erstreckenden Fortsatz 28 auf, der mit Nocken 19 der Antriebswelle 10 in Eingriff ist, indem er in einen zwischen zweien dieser Nocken 19 gebildeten Zwischenraum eindringt. Gleichzeitig bewirkt die Zugfeder 7, dass die beiden zweiten Hebelarme 18 aufeinander zu gezogen werden, so dass die beiden ersten Hebelarme 17 in radialer Richtung an das Schaltrohr 23 gedrückt werden. Hierdurch sind die Antriebswelle 10 und damit auch die Werkzeugaufnahme 1 gegenüber dem Gehäuse 13 gesperrt, sodass hier eine Sperrposition S vorliegt.

In Figur 6 ist das Schaltrohr 23 im Detail dargestellt. Dieses enthält die bereits erwähnte erste Rampe 20 sowie eine Öffnung 27, durch welche die Verschlussschraube 25 hindurch bis in den Schaltring 26 vordringt.

Der in Figur 7 im Detail dargestellte Sperrhebel 16 weist ausser dem bereits erwähnten ersten Hebelarm 17 mit der daran angeordneten zweiten Rampe 21 und dem zweiten Hebelarm 18 mit daran angeordnetem Fortsatz 28 noch einen Stift 29 auf, um den er verschwenkt werden kann.

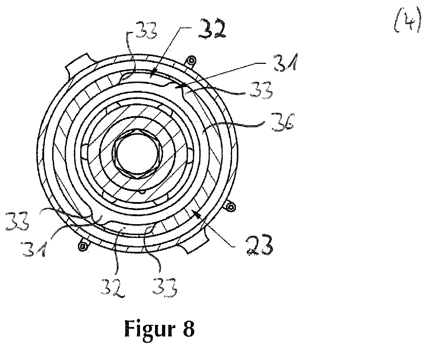

Das Schaltelement 2 ist derart ausgebildet und angeordnet, dass es nur dann relativ zum Gehäuse 13 verdrehbar ist, wenn die darauf einwirkende axiale Druckkraft den ersten Schwellenwert unterschreitet. Wie in Figur 8 gezeigt ist, sind an einem Lagerhalter 36 zwei radial nach aussen vorstehende Nocken 31 angeformt. Weiterhin ist das Schaltrohr 23 erkennbar, an dessen Innenseite zwei Aussparungen 32 gebildet sind, die jeweils in Umfangsrichtung von zwei Flanken 33 begrenzt werden. Die zwei Nocken 31 befinden sich in der Ruheposition 4 in jeweils einer der beiden Aussparungen 32. Hierdurch wird in der Ruheposition die Drehbewegung von Schaltelement 2 und Schaltrohr 23 innerhalb eines vorgegebenen Winkelbereichs begrenzt. Auf diese Weise kann in der Ruheposition zwischen beiden Drehrichtung umgeschaltet werden.

In der Ruheposition gemäss Figuren 1 bis 8 kann ein Benutzer, beispielsweise ein Chirurg, den Schraubendreher 100 am Gehäuse 13 ergreifen, ohne gleichzeitig das Schaltelement 2 zu ergreifen. Dabei kann er auch eine axiale Druckkraft auf den Schraubendreher 100 und damit auch auf die Werkzeugaufnahme 1 ausüben, ohne dass hierdurch die auf das Schaltelement 2 einwirkende axiale Druckkraft den ersten Schwellenwert überschreitet und somit die Freigabeposition F erreicht wird oder sogar die Werkzeugaufnahme 1 vom Elektromotor 9 angetrieben wird. Dies ist vor allem für den manuellen Modus besonders vorteilhaft, in dem beispielsweise eine Knochenschraube unter Aufbietung nicht nur einer Kraft in Umfangsrichtung, sondern auch einer axialen Druckkraft eingedreht werden soll.

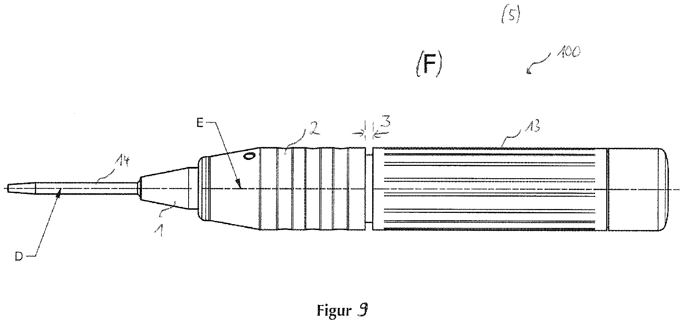

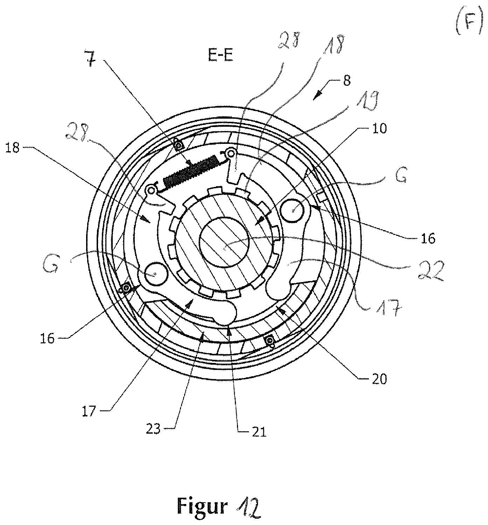

Wie bereits ausgeführt wurde, kann das Schaltelement 2 ausgehend von der in Figur 1 dargestellten Ruheposition 4 durch Ausüben einer axialen Druckkraft gegenüber dem Gehäuse 13 in axialer Richtung verschoben werden. Diese genannte axiale Druckkraft entsteht, wenn der Schraubendreher 100 am Schaltelement 2 gehalten wird und gegen einen Gegenstand gedrückt wird, beispielsweise gegen eine Knochenschraube. Durch Vergleich mit Figur 1 ist erkennbar, dass das Schaltelement 2 um den Schaltweg 3 bewegt wurde. Die hierdurch erreichte Aktivposition 5 ist in Figur 9 dargestellt. Dabei wurde zunächst bei Überschreiten des ersten Schwellenwertes die Sperrung zwischen Werkzeugaufnahme 1 und Gehäuse 13 gelöst, so dass die Freigabeposition F erreicht wurde (siehe dazu auch Figur 12), und anschliessend wurde bei Überschreiten auch des zweiten Schwellenwertes der Elektromotor 9 eingeschaltet (siehe dazu auch Figur 11).

Figur 10 zeigt eine Vorderansicht auf den Schraubendreher 100 in Richtung auf die Schraubendreherklinge 14. Dabei ist der Sperrhebel als verdeckte Kanten dargestellt. Eine Schnittansicht entlang der Linie B-B ist in Figur 11 enthalten. Diese Schnittebene ist gegenüber den in Figur 2 dargestellten Schnittebenen A-A und C-C verkippt und verläuft durch das Zentrum der zweiten Rampe 21, dargestellt in der Freigabeposition F.

Gegenüber Figur 4, die die Ruheposition 4 zeigt, ist hier aufgrund der axialen Bewegung des Schaltringes 26 der Schaltkontakt 11 in Kontakt mit zweien oder dreien der Kontaktstifte 12, in Abhängigkeit von der Drehstellung des Schaltelementes 2. Aufgrund des hierdurch erreichten Kontaktes wurde hier über nicht dargestellte, mit den Kontaktstiften 12 verbundene Drahtbrücken der Elektromotor 9 eingeschaltet, sodass die Werkzeugaufnahme 1 in Rotation versetzt wurde.

Bevor der zweite Schwellenwert der axialen Druckkraft überschritten ist und der genannte elektrische Kontakt entsteht, werden die Sperrmittel 8 bei Überschreiten des ersten Schwellenwertes von der Sperrposition S gemäss Figur 5 in die in Figur 12 dargestellte Freigabeposition F gebracht. Durch axiale Verschiebung des Schaltelementes 2 wird auch das Schaltrohr 23 in Richtung auf die Werkzeugaufnahme 1 verschoben. Durch Entlanggleiten der zweiten Rampen 21 der Sperrhebel 16 an der ersten Rampe 20 des Schaltrohres 23 werden die ersten Hebelarme 17 der Sperrhebel 16 gegen die Kraft der Zugfeder 7 in radialer Richtung nach innen gedrückt. Die Sperrhebel 16 werden folglich um ihre Schwenkachsen G verschwenkt, sodass die an den zweiten Hebelarmen 18 gebildeten Fortsätze 28 ausser Eingriff mit den Nocken 19 geraten. Auf diese Weise ist die Antriebswelle 10 gegenüber dem Gehäuse frei beweglich, sodass die Freigabeposition F vorliegt. Durch den genannten Mechanismus wird also die axiale Bewegung des Schaltrohres 23 in eine Schwenkbewegung der Sperrhebel 16 in einer im Wesentlichen senkrecht zur Drehachse verlaufenden Ebene umgesetzt.

Wie oben bereits erwähnt wurde, ist es natürlich auch denkbar, dass alternativ nur die erste Kontaktfläche 20 als Rampe ausgebildet ist und die zweite Kontaktfläche 21 als Ecke oder als sphärische Teilfläche, die an der Rampe 20 entlanggleiten kann, oder nur die zweite Kontaktfläche 21 ist als Rampe ausgebildet und die erste Kontaktfläche 20 als Ecke oder als sphärische Teilfläche, die an der Rampe 20 entlanggleiten kann.
Figur 13 zeigt entlang der in Figur 3 eingezeichneten Schnittlinie F-F die drei Kontaktstifte 12, die jeweils über hier nicht erkennbare Drahtbrücken mit dem Elektromotor 9 verbunden sind. In Figur 14, die eine Schnittansicht entlang der in Figur 3 eingezeichneten Schnittlinie G-G zeigt, ist der kreisbogenförmige Schaltkontakt 11 erkennbar. Dieser ist in einem Schaltring 26 aufgenommen, der mittels der Verschlussschrauben 25 starr mit dem Schaltelement 2 und dem Schaltrohr 23 verbunden ist. Durch Drehen des Schaltelementes 2 relativ zum Gehäuse 13 um die Drehachse D herum wird also auch der Schaltring 26 relativ zum Gehäuse 13 um die Drehachse D herum gedreht. Hierdurch ist der Schaltkontakt 11 abhängig von der Drehstellung entweder mit zweien oder dreien der Kontaktstifte 12 in Kontakt: In einer ersten Drehstellung des Schaltelementes 2 und bei Überschreiten des zweiten Schwellenwertes der axialen Druckkraft verbindet der Schaltkontakt 11 zwei der Kontaktstifte 12, wodurch die Werkzeugaufnahme 1 in einer ersten Drehrichtung angetrieben wird. In einer zweiten Drehstellung des Schaltelementes 2 und bei Überschreiten des zweiten Schwellenwertes der axialen Druckkraft verbindet der Schaltkontakt 11 alle drei Kontaktstifte 12 miteinander, sodass die Werkzeugaufnahme 1 in einer zweiten Drehrichtung angetrieben wird, welche zur ersten Drehrichtung entgegengesetzt ist.

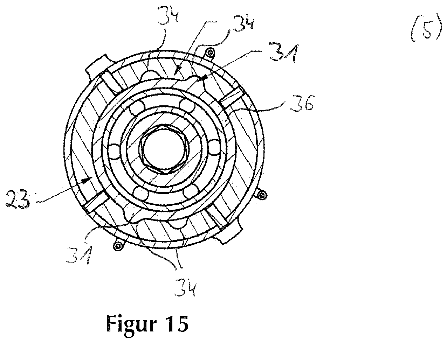

Figur 15 zeigt, wie in der Aktivposition 5 die Nocken 31 in an der Innenseite des Schaltrohres 23 gebildete Aussparungen 34 aufgenommen sind, wodurch die Drehbewegung blockiert wird. Hierdurch kann in dieser Aktivposition 5 nicht mehr zwischen den beiden Drehrichtungen umgeschaltet werden kann.

## Patentansprüche

1. Elektrische Werkzeugmaschine (100), insbesondere elektrischer Schraubendreher (100), insbesondere für die Verwendung in der Chirurgie, enthaltend
- ein Gehäuse (13);
- ein Rotationswerkzeug (14), insbesondere eine Schraubendreherklinge (14), oder eine Werkzeugaufnahme (1) zum insbesondere lösbaren Aufnehmen eines Rotationswerkzeugs (14), insbesondere einer Schraubendreherklinge (14);
- einen Elektromotor (9) zum Antreiben des Rotationswerkzeugs (14) bzw. der Werkzeugaufnahme (1);
- Sperrmittel (8), welche in eine Sperrposition (S) und in eine Freigabeposition (F) bringbar sind;
**gekennzeichnet durch**
mindestens einen Schalter (11, 12), welcher derart ausgebildet und angeordnet ist, dass das Rotationswerkzeug (14) bzw. die Werkzeugaufnahme (1) nur dann vom Elektromotor (9) antreibbar ist, wenn eine auf ein Betätigungselement (2) der Werkzeugmaschine (100) einwirkende axiale Druckraft einen vorgegebenen zweiten Schwellenwert überschreitet, wobei die Sperrmittel (8) derart ausgebildet und angeordnet sind, dass das Rotationswerkzeug (14) bzw. die Werkzeugaufnahme (1)
- in der Sperrposition (S) gegenüber dem Gehäuse (13) gesperrt oder nur bei Überschreiten eines vorgegebenen Drehmoments relativ zum Gehäuse (13) beweglich ist, wenn eine auf das Betätigungselement (2) einwirkende axiale Druckkraft einen vorgegebenen ersten Schwellenwert unterschreitet, und
- in der Freigabeposition (F) gegenüber dem Gehäuse (13) beweglich ist, wenn die auf das Betätigungselement (2) einwirkende axiale Druckkraft den vorgegebenen ersten Schwellenwert überschreitet.

2. Werkzeugmaschine (100) gemäss Anspruch 1,
**dadurch gekennzeichnet, dass**
das Betätigungselement (2) durch ein Schaltelement (2) gebildet ist, welches gegenüber dem Gehäuse (13) in axialer Richtung verschiebbar ist.

3. Werkzeugmaschine (100) gemäss Anspruch 2,
**dadurch gekennzeichnet, dass**
das Schaltelement (2) in axialer Richtung vorgespannt ist, insbesondere mittels mindestens einer Feder (6), insbesondere mittels mindestens einer Druckfeder (6), bevorzugt in einer vom Rotationswerkzeug (14) bzw. von der Werkzeugaufnahme (1) weg weisenden Richtung.

4. Werkzeugmaschine (100) gemäss einem der Ansprüche 2 oder 3,
**dadurch gekennzeichnet, dass**
das Schaltelement (2) derart ausgebildet und angeordnet ist, dass die Werkzeugmaschine (100) von einem Benutzer durch insbesondere einhändiges Halten nur am Schaltelement (2) bestimmungsgemäss bedienbar ist.

5. Werkzeugmaschine (100) gemäss einem der vorangehenden Ansprüche,
**dadurch gekennzeichnet, dass**
die Sperrmittel (8) derart vorgespannt sind, dass sie sich bei Unterschreiten des ersten Schwellenwertes in der Sperrposition (S) befinden.

6. Werkzeugmaschine (100) gemäss einem der vorangehenden Ansprüche,
**dadurch gekennzeichnet, dass**
sie ein insbesondere starr mit dem Betätigungselement (2) verbundenes Schaltrohr (23) aufweist, das bei Einwirken einer axialen Druckkraft auf das Betätigungselement (2) gegenüber dem Gehäuse (13) in axialer Richtung verschiebbar ist und nur bei Überschreiten des ersten Schwellenwertes derart mit den Sperrmitteln (8) zusammenwirkt, dass diese sich in der Freigabeposition (F) befinden.

7. Werkzeugmaschine (100) gemäss Anspruch 6,
**dadurch gekennzeichnet, dass**
die Sperrmittel (8) mindestens einen Sperrhebel (16) mit einem ersten Hebelarm (17) und einem zweiten Hebelarm (18) enthalten, wobei der Sperrhebel (16) derart mit dem Schaltrohr (23) zusammenwirkt, dass der Sperrhebel (16) nur bei Unterschreiten des ersten Schwellenwertes durch Eingriff des ersten Hebelarmes (17) mit dem Schaltrohr (23) in die Sperrposition (S) bewegbar ist, in der der zweite Hebelarm (18) mit mindestens einem Nocken (19) einer Antriebswelle (10) der Werkzeugmaschine (100) in Eingriff ist, die den Elektromotor (9) mit dem Rotationswerkzeug (14) bzw. der Werkzeugaufnahme (1) verbindet.

8. Werkzeugmaschine gemäss Anspruch 7,
**dadurch gekennzeichnet, dass**
das Schaltrohr (23) eine erste Kontaktfläche (20), insbesondere eine erste Rampe (20), aufweist und der erste Hebelarm (17) eine zweite Kontaktfläche (21), insbesondere eine zweite Rampe (21), aufweist, welche derart ausgebildet und angeordnet sind, dass der erste Hebelarm (17) bei Überschreiten des ersten Schwellenwertes durch Kontakt der zweiten Kontaktfläche (21) mit der ersten Kontaktfläche (20) in radialer Richtung bewegt wird.

9. Werkzeugmaschine (100) gemäss einem der vorangehenden Ansprüche,
**dadurch gekennzeichnet, dass**
der Elektromotor (9) und/oder eine den Elektromotor (9) mit dem Rotationswerkzeug (14) bzw. der Werkzeugaufnahme (1) verbindende Kupplung und/oder ein den Elektromotor (9) mit dem Rotationswerkzeug (14) bzw. der Werkzeugaufnahme (1) verbindendes Getriebe derart ausgebildet und/oder angeordnet ist, dass das Rotationswerkzeug (14) bzw. die Werkzeugaufnahme (1) wahlweise in einer von zwei zueinander entgegengesetzten Drehrichtungen antreibbar ist.

10. Werkzeugmaschine (100) gemäss einem der vorangehenden Ansprüche,
**dadurch gekennzeichnet, dass**
sie ein Schaltelement (2) aufweist, insbesondere ein Schaltelement (2) mit den Merkmalen eines der Ansprüche 2 bis 4, mit dessen Hilfe mindestens ein Antriebsparameter der Werkzeugmaschine (100), insbesondere die Drehrichtung und/oder die Drehgeschwindigkeit des Rotationswerkzeuges (14) bzw. der Werkzeugaufnahme (1), auswählbar ist, wobei das Schaltelement (2) relativ zum Gehäuse (13) um eine Einstellachse (E) verdrehbar ist, welche im Wesentlichen parallel zur Drehachse (D) des Rotationswerkzeugs (14) bzw. der Werkzeugaufnahme (1) verläuft oder damit übereinstimmt.

11. Werkzeugmaschine (100) gemäss Anspruch 10,
**dadurch gekennzeichnet, dass**
das Schaltelement (2) derart ausgebildet und angeordnet ist, dass es nur dann relativ zum Gehäuse (13) verdrehbar ist, wenn die auf das Betätigungselement (2), insbesondere auf das Schaltelement (2), einwirkende axiale Druckkraft den ersten Schwellenwert unterschreitet.

12. Werkzeugmaschine (100) gemäss einem der Ansprüche 10 und 11,
**dadurch gekennzeichnet, dass**
der Schalter (11, 12) mindestens einen Schaltkontakt (11) und mindestens einen, bevorzugt mindestens zwei, besonders bevorzugt drei Kontaktstifte (12) enthält, wobei der Schaltkontakt (11) in Abhängigkeit von der Stellung des Schaltelementes (2) mit dem Kontaktstift (12) bzw. mit einem oder mehreren der Kontaktstifte (12) verbindbar ist.

13. Werkzeugmaschine (100) gemäss einem der Ansprüche 10 bis 12,
**dadurch gekennzeichnet, dass**
der Schalter (11, 12) einen insbesondere kreisbogenförmigen Schaltkontakt (11) und mindestens drei Kontaktstifte (12) enthält, wobei der Schaltkontakt (11) bei Überschreiten des zweiten Schwellenwertes
- in einer ersten Stellung des Schaltelementes (2) einen ersten der Kontaktstifte (12) mit einem zweiten der Kontaktstifte (12) verbindet, so dass das Rotationswerkzeug (14) bzw. die Werkzeugaufnahme (1) in einer ersten Drehrichtung antreibbar ist, und
- in einer zweiten Stellung des Schaltelementes (2) den ersten der Kontaktstifte (12), den zweiten der Kontaktstifte (12) und einen dritten der Kontaktstifte (12) miteinander verbindet, so dass das Rotationswerkzeug (14) bzw. die Werkzeugaufnahme (1) in einer zweiten Drehrichtung antreibbar ist, welche zur ersten Drehrichtung entgegengesetzt ist.

14. Werkzeugmaschine (100) gemäss einem der vorangehenden Ansprüche,
**dadurch gekennzeichnet, dass**
der Schalter (11, 12) derart ausgebildet und angeordnet ist, dass der Elektromotor (9) mittels des Schalters (11, 12) eingeschaltet wird, wenn eine auf das Betätigungselement (2) einwirkende axiale Druckkraft den zweiten Schwellenwert überschreitet.

15. Werkzeugmaschine (100) gemäss einem der vorangehenden Ansprüche,
**dadurch gekennzeichnet, dass**
der erste Schwellenwert kleiner ist als der zweite Schwellenwert.

## Claims

1. Electric machine tool (100), in particular electric screw driver (100), in particular for use in surgery, containing
- a housing (13);
- a rotational tool (14), in particular a screw driver blade (14), or a tool receptacle (1), for, in particular, detachably accommodating a rotational tool (14), in particular a screw driver blade (14);
- an electric motor (9) for driving the rotational tool (14) and/or the tool receptacle (1);
- locking means (8) which can be moved into a locking position (S) and into a release position (F) ;
**characterized by**
at least one switch (11, 12) which is embodied and arranged in such a way that the rotational tool (14) and/or the tool receptacle (1) can be driven by the electric motor (9) only if an axial compressive force which acts on an activation element (2) of the machine tool (100) exceeds a predefined second threshold value, wherein the locking means (8) are embodied and arranged in such a way that the rotational tool (14) and/or the tool receptacle (1)
- in the locked position (S) is locked with respect to the housing (13) or is movable relative to the housing (13) only when a predefined torque is exceeded, if an axial compressive force acting on the activation element (2) falls below a predefined first threshold value, and
- in the release position (F) is movable relative to the housing (13) if the axial compressive force acting on the activation element (2) exceeds the predefined first threshold value.

2. Machine tool (100) according to Claim 1,
**characterized in that**
the activation element (2) is formed by a switching element (2) which is displaceable in an axial direction relative to the housing (13).

3. Machine tool (100) according to Claim 2,
**characterized in that**
the switching element (2) is prestressed in the axial direction, in particular by means of at least one spring (6), in particular by means of at least one compression spring (6), preferably in a direction pointing away from the rotational tool (14) or from the tool receptacle (1).

4. Machine tool (100) according to one of the Claims 2 or 3,
**characterized in that**
the switching element (2) is embodied and arranged in such a way that the machine tool (100) can be operated by a user in the approved fashion by, in particular, holding only the switching element (2) with one hand.

5. Machine tool (100) according to one of the preceding claims,
**characterized in that**
the locking means (8) are prestressed in such a way that when the first threshold value is undercut they are in the locked position (S).

6. Machine tool (100) according to one of the preceding claims,
**characterized in that**
said machine tool (100) has a switching tube (23) which is connected, in particular rigidly, to the activation element (2) and which, when an axial compressive force acts on the activation element (2), is displaceable in the axial direction relative to the housing (13), and only when the first threshold value is exceeded does said switching tube (23) interact with the locking means (8) in such a way that they are in the release position (F).

7. Machine tool (100) according to Claim 6,
**characterized in that**
the locking means (8) contain at least one locking lever (16) with a first lever arm (17) and a second lever arm (18), wherein the locking lever (16) interacts with the switching tube (23) in such a way that only when the first threshold value is undercut, the locking lever (16) is movable through engagement of the first lever arm (17) with the switching tube (23), into the locked position (S) in which the second lever arm (18) is in engagement with at least one cam (19) of a drive shaft (10) of the machine tool (100) which connects the electric motor (9) to the rotational tool (14) and/or the tool receptacle (1).

8. Machine tool according to Claim 7,
**characterized in that**
the switching tube (23) has a first contact surface (20), in particular a first ramp (20), and the first lever arm (17) has a second contact surface (21), in particular a second ramp (21), wherein said contact surfaces (20, 21) are embodied and arranged in such a way that, when the first threshold value is exceeded, the first lever arm (17) is moved in the radial direction through contact of the second contact surface (21) with the first contact surface (20).

9. Machine tool (100) according to one of the preceding claims,
**characterized in that**
the electric motor (9) and/or a coupling connecting the electric motor (9) to the rotational tool (14) or the tool receptacle (1) and/or a transmission connecting the electric motor (9) to the rotational tool (14) or the tool receptacle (1) are/is embodied and/or arranged in such a way that the rotational tool (14) or the tool receptacle (1) can selectively be driven in one of two rotational directions which are opposed to one another.

10. Machine tool (100) according to one of the preceding claims,
**characterized in that**
said machine tool (100) has a switching element (2), in particular a switching element (2) having the features of one of Claims 2 to 4, with the aid of which at least one drive parameter of the machine tool (100), in particular the rotational direction and/or the rotational speed of the rotational tool (14) or of the tool receptacle (1), can be selected, wherein the switching element (2) can be rotated relative to the housing (13) about an adjustment axis (E) which runs essentially parallel to the rotational axis (D) of the rotational tool (14) or of the tool receptacle (1) or corresponds thereto.

11. Machine tool (100) according to Claim 10,
**characterized in that**
the switching element (2) is embodied and arranged in such a way that it can be rotated relative to the housing (13) only if the axial compressive force acting on the activation element (2), in particular on the switching element (2), falls below the first threshold value.

12. Machine tool (100) according to one of Claims 10 or 11,
**characterized in that**
the switch (11, 12) contains at least one switching contact (11) and at least one, preferably at least two, particularly preferably three, contact pins (12), wherein the switching contact (11) can be connected to the contact pin (12) and/or to one or more of the contact pins (12) as a function of the position of the switching element (2).

13. Machine tool (100) according to one of Claims 10 to 12,
**characterized in that**
the switch (11, 12) contains an, in particular, circular-arc-shaped switching contact (11) and at least three contact pins (12), wherein the switching contact (11), when the second threshold value is exceeded,
- in a first position of the switching element (2), connects a first of the contact pins (12) to a second of the contact pins (12) so that the rotational tool (14) or the tool receptacle (1) can be driven in a first rotational direction, and
- in a second position of the switching element (2), the switching contact (11) connects the first of the contact pins (12), the second of the contact pins (12) and a third of the contact pins (12) to one another, so that the rotational tool (14) or the tool receptacle (1) can be driven in a second rotational direction which is opposed to the first rotational direction.

14. Machine tool (100) according to one of the preceding claims,
**characterized in that**
the switch (11, 12) is embodied and arranged in such a way that the electric motor (9) is switched on by means of the switch (11, 12) if an axial compressive force acting on the activation element (2) exceeds the second threshold value.

15. Machine tool (100) according to one of the preceding claims,
**characterized in that**
the first threshold value is lower than the second threshold value.

## Revendications

1. Machine-outil (100) électrique, notamment tournevis électrique (100), en particulier destinée à être utilisée en chirurgie, comprenant
- un boîtier (13) ;
- un outil rotatif (14), notamment une lame de tournevis (14), ou un attachement d'outil (1) destiné à accueillir, en particulier de manière amovible, un outil rotatif (14), notamment une lame de tournevis (14) ;
- un moteur électrique (9) destiné à entraîner l'outil rotatif (14) ou l'attachement d'outil (1) ;
- des moyens de blocage (8) qui peuvent être amenés dans une position de blocage (S) et dans une position de libération (F) ;
**caractérisée par**
au moins un commutateur (11, 12) qui est configuré et disposé de telle sorte que l'outil rotatif (14) ou l'attachement d'outil (1) ne peut être entraîné par le moteur électrique (9) que lorsqu'une force de pression axiale qui agit sur un élément d'actionnement (2) de la machine-outil (100) devient supérieure à une deuxième valeur de seuil prédéfinie, les moyens de blocage (8) étant configurés et disposés de telle sorte que l'outil rotatif (14) ou l'attachement d'outil (1),
- dans la position de blocage (S), est bloqué par rapport au boîtier (13) ou ne peut être déplacé par rapport au boîtier (13) qu'en cas de dépassement d'un couple prédéfini lorsqu'une force de pression axiale qui agit sur l'élément d'actionnement (2) devient inférieure à une première valeur de seuil prédéfinie, et
- dans la position de libération (F), peut être déplacé par rapport au boîtier (13) lorsque la force de pression axiale qui agit sur l'élément d'actionnement (2) devient supérieure à la première valeur de seuil prédéfinie.

2. Machine-outil (100) selon la revendication 1, **caractérisée en ce que** l'élément d'actionnement (2) est formé par un élément de commutation (2) qui peut être déplacé dans la direction axiale par rapport au boîtier (13) .

3. Machine-outil (100) selon la revendication 2, **caractérisée en ce que** l'élément de commutation (2) est précontraint dans le sens axial, notamment au moyen d'au moins un ressort (6), en particulier au moyen d'au moins un ressort presseur (6), de préférence dans une direction qui s'éloigne de l'outil rotatif (14) ou de l'attachement d'outil (1).

4. Machine-outil (100) selon l'une des revendications 2 ou 3, **caractérisée en ce que** l'élément de commutation (2) est configuré et disposé de telle sorte que la machine-outil (100) peut être utilisée conformément à sa destination par un utilisateur en la maintenant notamment d'une seule main seulement au niveau de l'élément de commutation (2).

5. Machine-outil (100) selon l'une des revendications précédentes, **caractérisée en ce que** les moyens de blocage (8) sont précontraints de telle sorte qu'ils se trouvent dans la position de blocage (S) en cas de franchissement vers le bas de la première valeur de seuil.

6. Machine-outil (100) selon l'une des revendications précédentes, **caractérisée en ce qu'**elle possède un tube de commutation (23) relié notamment de manière rigide à l'élément d'actionnement (2), lequel peut être déplacé dans le sens axial par rapport au boîtier (13) lorsqu'une force de pression axiale est exercée sur l'élément d'actionnement (2) et ne coopère avec les moyens de blocage (8) qu'en cas de dépassement de la première valeur de seuil de telle sorte que ceux-ci se trouvent dans la position de libération (F).

7. Machine-outil (100) selon la revendication 6, **caractérisée en ce que** les moyens de blocage (8) contiennent au moins un levier de blocage (16) muni d'un premier bras de levier (17) et d'un deuxième bras de levier (18), le levier de blocage (16) coopérant avec le tube de commutation (23) de telle sorte que le levier de blocage (16) ne peut être déplacé dans la position de blocage (S) par mise en prise du premier bras de levier (17) avec le tube de commutation (23) qu'en cas de franchissement vers le bas de la première valeur de seuil, position de blocage (S) dans laquelle le deuxième bras de levier (18) est en prise avec au moins une came (19) d'un arbre d'entraînement (10) de la machine-outil (100) qui relie le moteur électrique (9) à l'outil rotatif (14) ou à l'attachement d'outil (1) .

8. Machine-outil selon la revendication 7, **caractérisée en ce que** le tube de commutation (23) possède une première surface de contact (20), notamment une première rampe (20), et le premier bras de levier (17) possède une deuxième surface de contact (21), notamment une deuxième rampe (21), qui sont configurées et disposées de telle sorte que le premier bras de levier (17), en cas de dépassement de la première valeur de seuil, est déplacé dans la direction radiale par contact de la deuxième surface de contact (21) avec la première surface de contact (20).

9. Machine-outil (100) selon l'une des revendications précédentes, **caractérisée en ce que** le moteur électrique (9) et/ou un accouplement qui relie le moteur électrique (9) à l'outil rotatif (14) ou à l'attachement d'outil (1) et/ou un engrenage qui relie le moteur électrique (9) à l'outil rotatif (14) ou à l'attachement d'outil (1) est configuré et/ou disposé de telle sorte que l'outil rotatif (14) ou l'attachement d'outil (1) peut être entraîné au choix dans l'un parmi deux sens de rotation inverses.

10. Machine-outil (100) selon l'une des revendications précédentes, **caractérisée en ce qu'**elle possède un élément de commutation (2), notamment un élément de commutation (2) ayant les caractéristiques de l'une des revendications 2 à 4, à l'aide duquel au moins un paramètre d'entraînement de la machine-outil (100), en particulier le sens de rotation et/ou la vitesse de rotation de l'outil rotatif (14) ou de l'attachement d'outil (1), peut être sélectionné, l'élément de commutation (2) pouvant être tourné par rapport au boîtier (13) autour d'un axe de réglage (E) qui s'étend sensiblement en parallèle de l'axe de rotation (D) de l'outil rotatif (14) ou de l'attachement d'outil (1) ou coïncide avec celui-ci.

11. Machine-outil (100) selon la revendication 10, **caractérisée en ce que** l'élément de commutation (2) est configuré et disposé de telle sorte qu'il ne peut tourner par rapport au boîtier (13) que lorsque la force de pression axiale qui agit sur l'élément d'actionnement (2), notamment sur l'élément de commutation (2), devient inférieure à la première valeur de seuil.

12. Machine-outil (100) selon l'une des revendications 10 et 11, **caractérisée en ce que** le commutateur (11, 12) contient au moins un contact de commutation (11) et au moins un, de préférence au moins deux, notamment de préférence trois pivots de contact (12), le contact de commutation (11) pouvant être relié au pivot de contact (12) ou à un ou plusieurs des pivots de contact (12) en fonction de la position de l'élément de commutation (2).

13. Machine-outil (100) selon l'une des revendications 10 à 12, **caractérisée en ce que** le commutateur (11, 12) contient un contact de commutation (11) notamment en forme d'arc de cercle et au moins trois pivots de contact (12), le contact de commutation (11), en cas de dépassement de la deuxième valeur de seuil
- dans une première position de l'élément de commutation (2), relie un premier des pivots de contact (12) à un deuxième des pivots de contact (12) de sorte que l'outil rotatif (14) ou l'attachement d'outil (1) peut être entraîné dans un premier sens de rotation et
- dans une deuxième position de l'élément de commutation (2), relie le premier des pivots de contact (12), le deuxième des pivots de contact (12) et un troisième des pivots de contact (12) ensemble de sorte que l'outil rotatif (14) ou l'attachement d'outil (1) peut être entraîné dans un deuxième sens de rotation, qui est l'inverse du premier sens de rotation.

14. Machine-outil (100) selon l'une des revendications précédentes, **caractérisée en ce que** le commutateur (11, 12) est configuré et disposé de telle sorte que le moteur électrique (9) est allumé au moyen du commutateur (11, 12) lorsqu'une force de pression axiale qui agit sur l'élément d'actionnement (2) devient supérieure à la deuxième valeur de seuil.

15. Machine-outil (100) selon l'une des revendications précédentes, **caractérisée en ce que** la première valeur de seuil est inférieure à la deuxième valeur de seuil.
